(19) Europäisches Patentamt
European Patent Office
Office européen des brevets

(11) **EP 4 024 407 A1**

(12) **EUROPEAN PATENT APPLICATION**

(43) Date of publication:
**06.07.2022 Bulletin 2022/27**

(21) Application number: **21175249.8**

(22) Date of filing: **21.05.2021**

(51) International Patent Classification (IPC):
**G16H 50/20** (2018.01) **G16H 50/70** (2018.01)

(52) Cooperative Patent Classification (CPC):
**G16H 50/70; G16H 50/20;** G16H 10/40;
G16H 10/60

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.12.2020 TW 109146620**

(71) Applicants:
• **National Health Research Institutes
Zhunan Town
350 Miaoli County (TW)**
• **Chang Gung Memorial Hospital, Keelung
Anle Dist. 20401 Keelung City (TW)**
• **Acer Healthcare Inc.
New Taipei City 22181 (TW)**
• **Acer Incorporated
New Taipei City 221 (TW)**

(72) Inventors:
• **Li, Chun-Hsien
221 New Taipei City (TW)**
• **Tsai, Tsung-Hsien
221 New Taipei City (TW)**
• **Hsu, Wei-Che
350 Zhunan Town (TW)**
• **Tsai, Ting-Fen
350 Zhunan Town (TW)**
• **Juang, Jyh-Lyh
350 Zhunan Town (TW)**
• **Yeh, Chi-Hsiao
20401 Keelung City (TW)**

(74) Representative: **2K Patentanwälte Blasberg
Kewitz & Reichel
Partnerschaft mbB
Schumannstrasse 27
60325 Frankfurt am Main (DE)**

Remarks:
Amended claims in accordance with Rule 137(2)
EPC.

(54) **ELECTRONIC DEVICE AND METHOD FOR SCREENING FEATURES FOR PREDICTING PHYSIOLOGICAL STATE**

(57)     An electronic device (100) and a method for screening features for predicting a physiological state are provided. The method includes: obtaining multiple physiological data corresponding to multiple features; generating multiple first subsets of the multiple features according to the multiple physiological data based on a first model, wherein the multiple first subsets respectively correspond to the multiple physiological data; selecting a first feature from the multiple features according to the multiple first subsets, calculating a first relation index of the first feature and a second feature corresponding to the multiple features, and selecting the second feature as an accompanied feature of the first feature according to the first relation index; and outputting the first feature and the accompanied feature.

FIG. 4

**Description**

BACKGROUND

Technical Field

The disclosure relates to an electronic device and a method for screening features for predicting a physiological state.

Description of Related Art

The use of blood to obtain multiple metabolite indexes of a test subject such that the doctor may determine the physiological state of the test subject according to the multiple metabolite indexes is an important medical technology that is gradually developing. Taking the determination of the degree of obesity of the test subject as an example, the conventional technology may use the body mass index (BMI) to determine the degree of obesity of the test subject, but the calculation of the BMI only takes into account the weight and height without considering the metabolic status of the test subject.

There are many types of metabolites produced by the human body, and the correlation between each metabolite and different physiological states is also different. Therefore, if a method for screening out metabolites that are highly related to a specific physiological state can be developed, the method may assist the doctor in monitoring the physiological state of the test subject more accurately.

SUMMARY

The disclosure provides an electronic device and a method for screening features for predicting a physiological state, which can screen out the features that are significantly related to a specific physiological state from multiple features.

An electronic device for screening features for predicting a physiological state of the disclosure includes a processor, a storage medium, and a transceiver. The storage medium stores multiple modules. The processor is coupled to the storage medium and the transceiver, and accesses and executes the multiple modules. The multiple modules include a data collection module, a training module, a computing module, and an output module. The data collection module obtains multiple physiological data corresponding to multiple features through the transceiver. The training module generates multiple first subsets of the multiple features according to the multiple physiological data based on a first model. The multiple first subsets correspond to the multiple physiological data. The computing module selects a first feature from the multiple features according to the multiple first subsets, calculates a first relation index of the first feature and a second feature corresponding to the multiple features, and selects the second feature as an accompanied feature of the first feature according to the first relation index. The output module outputs the first feature and the accompanied feature through the transceiver.

In an embodiment of the disclosure, the training module generates multiple second subsets of the multiple features according to the multiple physiological data based on a second model. The multiple second subsets respectively correspond to the multiple physiological data. The computing module selects the first feature from the multiple features according to the multiple first subsets and the multiple second subsets.

In an embodiment of the disclosure, the computing module calculates a first number of the first feature in the multiple first subsets, and calculates a second number of the first feature in the multiple second subsets. The computing module selects the first feature from the multiple features according to the first number and the second number.

In an embodiment of the disclosure, the computing module calculates a first score of the first feature according to the first number, a first weight corresponding to the first model, the second number, and a second weight corresponding to the second model. The computing module selects the first feature from the multiple features in response to the first score being greater than a first threshold value.

In an embodiment of the disclosure, the computing module calculates a first score of the first feature according to the first number, a first weight corresponding to the first model, the second number, and a second weight corresponding to the second model. The computing module calculates a third number of a third feature in the multiple first subsets and a fourth number of the third feature in the multiple second subsets. The computing module calculates a second score of the third feature according to the third number, the first weight, the fourth number, and the second weight. The computing module selects the first feature from the first feature and the third feature in response to the first score being greater than the second score.

In an embodiment of the disclosure, the computing module obtains a first number of the first feature in each of the multiple first subsets to generate a first vector. The computing module obtains a second number of the second feature in each of the multiple first subsets to generate a second vector. The computing module calculates the first relation index according to the first vector and the second vector.

In an embodiment of the disclosure, the computing module selects the second feature as the accompanied feature of the first feature in response to the first relation index being greater than a second threshold value.

In an embodiment of the disclosure, the computing module calculates a second relation index corresponding to a third feature and a fourth feature in the multiple features. The computing module selects the second feature as the accompanied feature of the first feature in response to the first relation index being greater than the second relation index.

In an embodiment of the disclosure, the training module trains at least one first prediction model of the physiological state according to the multiple physiological data, the first feature, and the accompanied feature, and calculates at least one first performance index corresponding to the at least one first prediction model. The training module randomly selects a third feature and a fourth feature from the multiple features. Any one of the third feature and the fourth feature is different from any one of the first feature and the second feature. The training module trains at least one second prediction model of the physiological state according to the multiple physiological data, the third feature, and the fourth feature, and calculates at least one second performance index of the at least one second prediction model. The computing module determines that the first feature and the accompanied feature are usable in response to the at least one first performance index being greater than the at least one second performance index. The output module outputs the first feature and the accompanied feature in response to the first feature and the accompanied feature being usable.

In an embodiment of the disclosure, the multiple features correspond to multiple metabolites of a human body.

In an embodiment of the disclosure, the data collection module receives a physiological data set through the transceiver, and divides the physiological data set into multiple training data and multiple test data respectively corresponding to the multiple physiological data according to a bootstrap. The training module generates the multiple first subsets according to the multiple training data. The training module generates the at least one first prediction model according to the multiple training data. The training module calculates the at least one first performance index according to the multiple test data.

In an embodiment of the disclosure, the first model or the second model is associated with one of a random forest algorithm, a logistic regression, and a support vector machine.

In an embodiment of the disclosure, the first model generates the multiple first subsets based on one of a stepwise selection and a feature importance.

A method for screening features for predicting a physiological state of the disclosure includes the following steps. Multiple physiological data corresponding to multiple features are obtained. Multiple first subsets of the multiple features are generated according to the multiple physiological data based on a first model. The multiple first subsets respectively correspond to the multiple physiological data. A first feature is selected from the multiple features according to the multiple first subsets, a first relation index of the first feature and a second feature corresponding to the multiple features is calculated, and the second feature is selected as an accompanied feature of the first feature according to the first relation index. The first feature and the accompanied feature are output.

Based on the above, the disclosure may select the feature that can significantly affect the prediction result of the physiological state of a test subject, and select the accompanied feature corresponding to the feature. The disclosure may output the feature and the accompanied feature as reference for the user. For example, assuming that the user is a doctor, the user may determine the degree of obesity of the test subject only by referring to the metabolite and the accompanied metabolite output by the disclosure without the need to spend effort on analyzing metabolites that are totally unrelated to obesity.

BRIEF DESCRIPTION OF THE DRAWINGS

FIG. 1 is a schematic diagram of an electronic device for screening features for predicting a physiological state according to an embodiment of the disclosure.
FIG. 2 is a flowchart of a method for screening features for predicting a physiological state according to an embodiment of the disclosure.
FIG. 3 is a flowchart of a method for determining whether a selected feature and an accompanied feature are usable according to an embodiment of the disclosure.
FIG. 4 is a flowchart of a method for screening features for predicting a physiological state according to another embodiment of the disclosure.

DETAILED DESCRIPTION OF DISCLOSED EMBODIMENTS

In order for the content of the disclosure to be more understandable, the following embodiments are specifically cited as examples on which the disclosure can be implemented. In addition, wherever possible, elements/components/steps with the same reference numerals in the drawings and embodiments represent the same or similar parts.

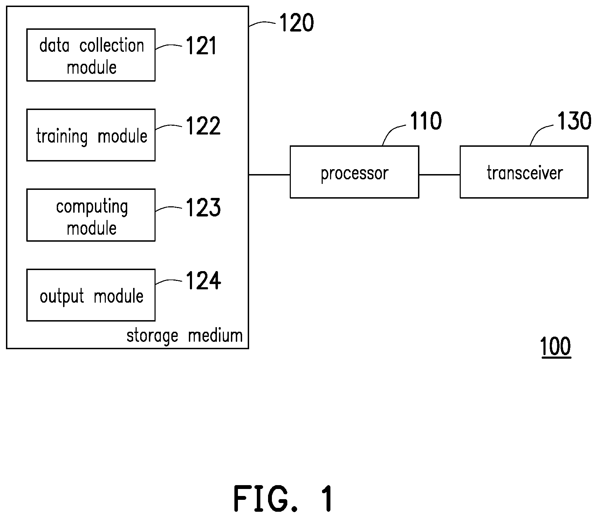

FIG. 1 is a schematic diagram of an electronic device 100 for screening features for predicting a physiological state according to an embodiment of the disclosure. The electronic device 100 may include a processor 110, a storage medium 120, and a transceiver 130.

The processor 110 is, for example, a central processing unit (CPU), other programmable general-purpose or specific-purpose micro control unit (MCU), microprocessor, digital signal processor (DSP), programmable controller, application specific integrated circuit (ASIC), graphics processing unit (GPU), image signal processor (ISP), image processing unit (IPU), arithmetic logic unit (ALU), complex programmable logic device (CPLD), field programmable gate array (FPGA), other similar elements, or a combination of the above elements. The processor 110 may be coupled to the storage medium 120 and the transceiver 130, and access and execute multiple modules and various applications stored in the storage medium 120.

The storage medium 120 is, for example, any type of fixed or removable random access memory (RAM), read-only memory (ROM), flash memory, hard disk drive (HDD), solid state drive (SSD), similar elements, or a combination of the above elements, which is used to store multiple modules or various applications that may be executed by the processor 110. In this embodiment, the storage medium 120 may store multiple modules including a data collection module 121, a training module 122, a computing module 123, an output module 124, etc., and the functions thereof will be explained later.

The transceiver 130 transmits and receives signals in a wireless or wired manner. The transceiver 130 may also execute operations such as low noise amplification, impedance matching, frequency mixing, up or down frequency conversion, filtering, and amplification.

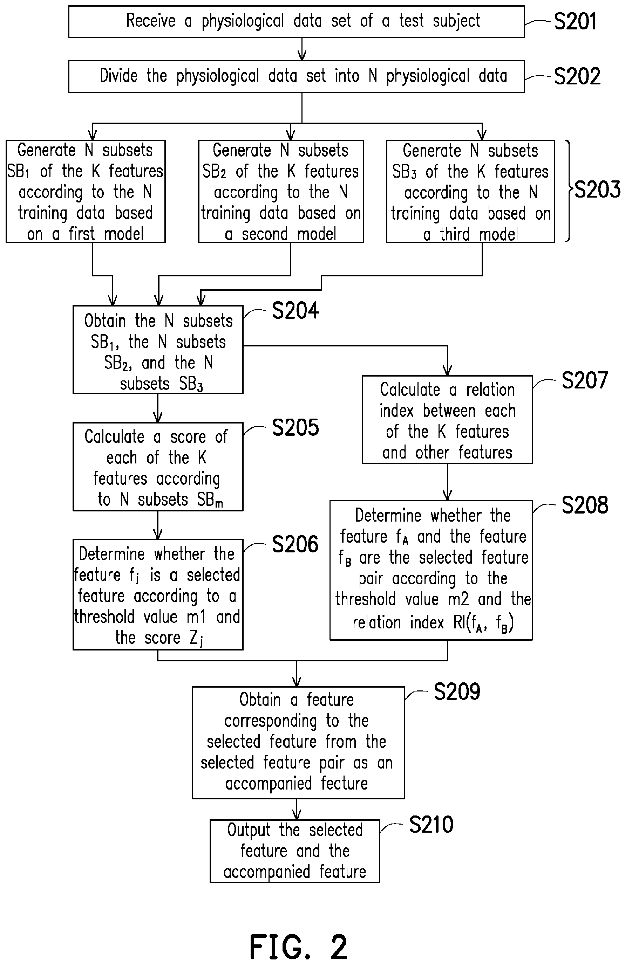

FIG. 2 is a flowchart of a method for screening features for predicting a physiological state according to an embodiment of the disclosure. The method may be implemented by the electronic device 100 shown in FIG. 1.

In Step S201, the data collection module 121 may receive a physiological data set of a test subject through the transceiver 130. The physiological data set may include K feature values respectively corresponding to K features. The K features (which are respectively features $f_1$, $f_2$, ..., $f_K$) may respectively correspond to K types of metabolites of a human body, where K may be any positive integer.

In Step S202, the data collection module 121 may divide the physiological data set into N physiological data corresponding to the K features, where N may be any positive integer. Specifically, the data collection module 121 may divide the physiological data set into the N physiological data according to a bootstrap. Each of the N physiological data may include training data and test data. In other words, the data collection module 121 may divide the physiological data set into N training data and N test data respectively corresponding to the N physiological data.

In an embodiment, the data collection module 121 may divide the physiological data set into a single physiological data corresponding to the K features. The physiological data may include the training data and the test data. Specifically, the data collection module 121 may divide the physiological data set into the training data and the test data according to k-fold cross-validation, thereby generating the physiological data.

In Step S203, the training module 122 may generate N subsets $SB_1$ of the K features according to the N training data based on a first model. The N subsets $SB_1$ may respectively correspond to the N training data. The first model may be configured to select one or more features that significantly affect a specific physiological state (for example, the degree of obesity) from the K features according to a training data. The one or more features are the subsets $SB_1$ of the K features. Accordingly, the N training data may generate the N subsets $SB_1$ of the K features, which are respectively subsets $SB_1^1$, $SB_1^2$, ..., $SB_1^N$.

Similarly, the training module 122 may generate N subsets $SB_2$ of the K features according to the N training data based on a second model. The N subsets $SB_2$ may respectively correspond to the N training data. The second model may be configured to select one or more features that significantly affect a specific physiological state (for example, the degree of obesity) from the K features according to a training data. The one or more features are the subsets $SB_2$ of the K features. Accordingly, the N training data may generate the N subsets $SB_2$ of the K features, which are respectively subsets $SB_2^1$, $SB_2^2$, ..., $SB_2^N$.

Similarly, the training module 122 may generate N subsets $SB_3$ of the K features according to the N training data based on a third model. The N subsets $SB_3$ may respectively correspond to the N training data. The third model may be configured to select one or more features that significantly affect a specific physiological state (for example, the degree of obesity) from the K features according to a training data. The one or more features are the subsets $SB_3$ of the K features. Accordingly, the N training data may generate the N subsets $SB_3$ of the K features, which are respectively subsets $SB_3^1$, $SB_3^2$, ..., $SB_3^N$.

A number M of models adopted in Step S203 may be defined by the user according to requirements. Although the number M in this embodiment is equal to 3 (that is, 3 models such as the first model, the second model, and the third model are adopted), the disclosure is not limited thereto. For example, the number M may be any positive integer greater than 1

In an embodiment, the first model (the second model, or the third model) may correspond to a random forest (RF) algorithm, a logistic regression, or a support vector machine (SVM), but the disclosure is not limited thereto. The first model (the second model, or the third model) may, for example, use a stepwise selection or a feature importance to select one or more features that significantly affect a specific physiological state from the K features. For example, the first model may use the stepwise selection to select one or more features from the K features based on a p-value or an Akaike information criterion (AIC). Different models may adopt the same or different algorithms. For example, the first model, the second model, and the third model may adopt the same or different algorithms.

In Step S204, the computing module 123 may obtain the N subsets $SB_1$ corresponding to the first model (which are respectively the subsets $SB_1^1$, $SB_1^2$, ..., $SB_1^N$) the N subsets $SB_2$ corresponding to the second model (which are respectively the subsets $SB_2^1$, $SB_2^2$, ..., $SB_2^N$), and the N subsets $SB_3$ corresponding to the third model (which are respectively the subsets $SB_3^1$, $SB_3^2$, ..., $SB_3^N$).

In Step S205, the computing module 123 may calculate a score of each of the K features according to N subsets $SB_m$, where m is the index of the model. For example, m=1 corresponds to the first model, m=2 corresponds to the second model, and m=3 corresponds to the third model.

Assuming that the computing module 123 intends to calculate a score $Z_j$ of a feature $f_j$ in the K features, the computing module 123 may calculate a number $S_{j,SB_m}$ of the feature $f_j$ in N the subsets $SB_m$ according to Equation (1), where $S_{j,SB_m}^i$ is the number of the feature $f_j$ in an i-th subset $SB_m$ in the N subsets $SB_m$ (and $S_{j,SB_m}^i$ may be 0 or 1).

$$S_{j,SB_m} = \sum_{i=1}^{N} S_{j,SB_m}^i \ldots (1)$$

For example, assuming that the total number of physiological data is 3 (N=3) and the total number of features is 5 (K=5), the computing module 123 may generate the following Table 1, Table 2, and Table 3 according to Equation (1). Table 1 corresponds to the first model, Table 2 corresponds to the second model, and Table 3 corresponds to the third model. Taking a feature $f_1$ in Table 1 as an example, a number $S_{1,SB_1}$ corresponding to the feature $f_1$ is $S_{1,SB_1} =$

$$S_{1,SB_1}^1 + S_{1,SB_1}^2 + S_{1,SB_1}^3 = 1 + 1 + 0 = 2.$$

Table 1

| Feature $f_j$ | 1st statistical result $S_{j,SB_1}^1$ of first model | 2nd statistical result $S_{j,SB_1}^2$ of first model | 3rd statistical result $S_{j,SB_1}^3$ of first model |
|---|---|---|---|
| Feature $f_1$ | 1 | 1 | 0 |
| Feature $f_2$ | 1 | 0 | 0 |
| Feature $f_3$ | 0 | 0 | 1 |
| Feature $f_4$ | 0 | 0 | 1 |

(continued)

| Feature $f_j$ | 1st statistical result $S^1_{j,SB_1}$ of first model | 2nd statistical result $S^2_{j,SB_1}$ of first model | 3rd statistical result $S^3_{j,SB_1}$ of first model |
|---|---|---|---|
| Feature $f_5$ | 0 | 1 | 0 |

Table 2

| Feature $f_j$ | 1st statistical result $S^1_{j,SB_2}$ of second model | 2nd statistical result $S^2_{j,SB_2}$ of second model | 3rd statistical result $S^3_{j,SB_2}$ of second model |
|---|---|---|---|
| Feature $f_1$ | 1 | 1 | 0 |
| Feature $f_2$ | 1 | 0 | 0 |
| Feature $f_3$ | 1 | 1 | 0 |
| Feature $f_4$ | 0 | 1 | 0 |
| Feature $f_5$ | 0 | 0 | 1 |

Table 3

| Feature $f_j$ | 1st statistical result $S^1_{j,SB_3}$ of third model | 2nd statistical result $S^2_{j,SB_3}$ of third model | 3rd statistical result $S^3_{j,SB_3}$ of third model |
|---|---|---|---|
| Feature $f_1$ | 1 | 1 | 0 |
| Feature $f_2$ | 0 | 1 | 0 |
| Feature $f_3$ | 0 | 0 | 1 |
| Feature $f_4$ | 1 | 0 | 0 |
| Feature $f_5$ | 1 | 1 | 0 |

After obtaining the number $S_{j,SB_m}$ of the feature $f_j$ in the N subsets $SB_m$, the computing module 123 may calculate a ratio $R_{j,SB_m}$ corresponding to the number $S_{j,SB_m}$ according to Equation (2), where $S_{j,SB_m}$ is the number corresponding to the N subsets $SB_m$ and the feature $f_j$.

$$R_{j,SB_m} = S_{j,SB_m}/N \ldots (2)$$

For example, assuming that N=3, the computing module 123 may generate the following Table 4 according to Table 1, Table 2, and Table 3 based on Equation (2). A number $S_{j,SB_1}$ and a ratio $R_{j,SB_1}$ correspond to the first model, a number $S_{j,SB_2}$ and a ratio $R_{j,SB_2}$ correspond to the second model, and a number $Sj_{,SB_3}$ and a ratio $R_{j,SB_3}$ correspond to the third model.

Table 4

| Feature $f_j$ | Number $S_{j,SB_1}$ | Number $S_{j,SB_2}$ | Number $S_{j,SB_3}$ | Ratio $R_{j,SB_1}$ | Ratio $R_{j,SB_2}$ | Ratio $R_{j,SB_3}$ |
|---|---|---|---|---|---|---|
| Feature $f_1$ | 2 | 2 | 2 | 2/3 | 2/3 | 2/3 |
| Feature $f_2$ | 1 | 1 | 1 | 1/3 | 1/3 | 1/3 |
| Feature $f_3$ | 1 | 2 | 1 | 1/3 | 2/3 | 1/3 |
| Feature $f_4$ | 1 | 1 | 1 | 1/3 | 1/3 | 1/3 |
| Feature $f_5$ | 1 | 1 | 2 | 1/3 | 1/3 | 2/3 |

After obtaining the ratio $R_{j,SB_m}$, the computing module 123 may calculate the score $Z_j$ corresponding to the feature $f_j$ according to Equation (3), where $w_m$ is the weight corresponding to an m-th model, and $R_{j,SB_m}$ is the ratio corresponding to the feature $f_j$ and the m-th model. For example, weights corresponding to the first model, the second model, and the third model may respectively be a weight $w_1 = 0$, a weight $w_2 = 0$, and a weight $w_3 = 1$. For another example, the weights corresponding to the first model, the second model, and the third model may respectively be the weight $w_1 = 1/3$, the weight $w_2 = 1/3$, and the weight $w_3 = 1/3$.

$$Z_j = \sum_{m=1}^{M} R_{j,SB_m} \cdot w_m \ldots (3)$$

For example, assuming that the weight $w_1 = 1/3$, the weight $w_2 = 1/3$, and the weight $w_3 = 1/3$, the computing module 123 may generate the following Table 5 according to Table 4 based on Equation (3) as shown below.

Table 5

| Feature $f_j$ | Ratio $R_{j,SB_1}$ | Ratio $R_{j,SB_2}$ | Ratio $R_{j,SB_3}$ | Score $Z_j$ |
|---|---|---|---|---|
| Feature $f_1$ | 2/3 | 2/3 | 2/3 | 2/3 |
| Feature $f_2$ | 1/3 | 1/3 | 1/3 | 1/3 |
| Feature $f_3$ | 1/3 | 2/3 | 1/3 | 4/9 |
| Feature $f_4$ | 1/3 | 1/3 | 1/3 | 1/3 |
| Feature $f_5$ | 1/3 | 1/3 | 2/3 | 4/9 |

After obtaining the score $Z_j$ corresponding to the feature $f_j$ in the K features, in Step S206, the computing module 123 may determine whether the feature $f_j$ is a selected feature according to a threshold value m1 and the score $Z_j$.
In an embodiment, the threshold value m1 may be associated with a score ranking of the feature $f_j$ in the K features. For example, the threshold value m1 may indicate that features with what high scores in the K features are used as selected features. Taking Table 5 as an example, the computing module 123 may select the feature $f_1$ with the highest score from the feature $f_1$ to the feature $f_5$ as the selected feature according to the threshold value m1. In other words, the computing module 123 may select the feature $f_1$ corresponding to the score $Z_1$ from the feature $f_1$ to the feature $f_5$ as the selected feature in response to the score $Z_1$ being greater than the score $Z_2$, the score $Z_3$, the score $Z_4$, and the score $Z_5$.
In an embodiment, the computing module 123 may select the feature $f_j$ as the selected feature in response to the score $Z_j$ exceeding the threshold value m1. Taking Table 5 as an example, assuming that the threshold value m1 is equal to 5/9, the computing module 123 may select the feature $f_1$ as the selected feature in response to the score $Z_1$ of the feature $f_1$ being greater than 5/9.
In Step S207, the computing module 123 may calculate a relation index between each of the K features and other features. Specifically, the computing module 123 may obtain K vectors respectively corresponding to the K features, and select two vectors from the K vectors to calculate the relation index between the two vectors.
If the computing module 123 intends to calculate the relation index between a feature $f_A$ and a feature $f_B$ in the K features, the computing module 123 may generate a vector $V_{A,SB_m}$ as shown in Equation (4) according to the number of the feature $f_A$ in the N subsets $SB_m$, and generate a vector $V_{B,SB_m}$ as shown in Equation (5) according to the number of the feature $f_B$ in the N subsets $SB_m$, where $S^i_{A,SB_m}$ is the number of the feature $f_A$ in the i-th subset in the N subsets $SB_m$, and $S^i_{B,SB_m}$ is the number of the feature $f_B$ in the i-th subset in the N subsets $SB_m$. Then, the computing module 123 may calculate the relation index between the feature $f_A$ and the feature $f_B$ according to the vector $V_{A,SB_m}$ and the vector $V_{B,SB_m}$. The relation index corresponds to the m-th model. The relation index is, for example, related to Pearson coefficient of correlation (PCC), but the disclosure is not limited thereto.

$$V_{A,SB_m} = (S^1_{A,SB_m}, S^2_{A,SB_m}, ..., S^N_{A,SB_m})...(4)$$

$$V_{B,SB_m} = (S^1_{B,SB_m}, S^2_{B,SB_m}, ..., S^N_{B,SB_m})...(5)$$

For example, the computing module 123 may generate the following Table 6 according to Tables 1, 2, and 3 based on Equation (4) and Equation (5). Table 6 includes M*K vectors corresponding to the K features (where K=5) and M models (where M=3). Each vector may include N elements (where N=3) corresponding to the N training data.

Table 6

| Feature $f_j$ | Vector $V_{j,SB_1}$ | Vector $V_{j,SB_2}$ | Vector $V_{j,SB_3}$ |
|---|---|---|---|
| Feature $f_1$ | $V_{1,SB_1} = (1,1,0)$ | $V_{1,SB_2} = (1,1,0)$ | $V_{1,SB_3} = (1,1,0)$ |
| Feature $f_2$ | $V_{2,SB_1} = (1,0,0)$ | $V_{2,SB_2} = (1,0,0)$ | $V_{2,SB_3} = (0,1,0)$ |
| Feature $f_3$ | $V_{3,SB_1} = (0,0,1)$ | $V_{3,SB_2} = (1,1,0)$ | $V_{3,SB_3} = (0,0,1)$ |
| Feature $f_4$ | $V_{4,SB_1} = (0,0,1)$ | $V_{4,SB_2} = (0,1,0)$ | $V_{4,SB_3} = (1,0,0)$ |
| Feature $f_5$ | $V_{5,SB_1} = (0,1,0)$ | $V_{5,SB_2} = (0,0,1)$ | $V_{5,SB_3} = (1,1,0)$ |

The computing module 123 may calculate the relation index corresponding to at least two features. For example, if the at least two features include only two features, the computing module 123 may calculate the relation index between the two features (for example, the feature $f_A$ and the feature $f_B$) based on Equation (6), where $C(V_x,V_y)$ is the correlation coefficient between a vector $V_x$ and a vector $V_y$, and $W_m$ is the weight corresponding to the m-th model. For another example, if the at least two features exceed two features, the computing module 123 may calculate the p-value of the at least two features based on an analysis of variance (ANOVA) test as the relation index.

$$RI(f_A, f_B) = \sum_{m=1}^{M} C(V_{A,SB_m}, V_{B,SB_m}) \cdot W_m...(6)$$

In an embodiment, the weights corresponding to the first model, the second model, and the third model may respectively be the weight $w_1 = 0$, the weight $w_2 = 0$, and the weight $w_3 = 1$. In an embodiment, the weights corresponding to the first model, the second model, and the third model may respectively be the weight $w_1 = 1/3$, the weight $w_2 = 1/3$, and the weight $w_3 = 1/3$. For example, if the weight $w_1 = 1/3$, the weight $w_2 = 1/3$, and the weight $w_3 = 1/3$, the computing module 123 may generate Table 7 according to the vectors of Table 6 based on Equation (6).

Table 7

| Feature pair | Correlation coefficient corresponding to first model | Correlation coefficient corresponding to second model | Correlation coefficient corresponding to third model | Relation index RI |
|---|---|---|---|---|
| $f_1, f_2$ | $C(V_{1,SB_1} V_{2,SB_1}) = 0.5$ | $C(V_{1,SB_2}, V_{2,SB_2}) = 0.5$ | $C(V_{1,SB_3}, V_{2,SB_3}) = 0.5$ | 1/2 |
| $f_1, f_3$ | $C(V_{1,SB_1}, V_{3,SB_3}) = -1.0$ | $C(V_{1,SB_2}, V_{3,SB_2}) = 1.0$ | $C(V_{1,SB_3}, V_{3,SB_3}) = -1$ | -1/3 |
| $f_1, f_4$ | $C(V_{1,SB_1}, V_{4,SB_1}) = -1.0$ | $C(V_{1,SB_2}, V_{4,SB_2}) = 0.5$ | $C(V_{1,SB_3}, V_{4,SB_3}) = 0.5$ | 0 |
| $f_1, f_5$ | $C(V_{1,SB_1} V_{5,SB_1}) = 0.5$ | $C(V_{1,SB_2}, V_{5,SB_2}) = -1.0$ | $C(V_{1,SB_3}, V_{5,SB_3}) = 1.0$ | 1/6 |

(continued)

| Feature pair | Correlation coefficient corresponding to first model | Correlation coefficient corresponding to second model | Correlation coefficient corresponding to third model | Relation index RI |
|---|---|---|---|---|
| $f_2, f_3$ | $C(V_{2,SB_1}, V_{3,SB_1}) = -0.5$ | $C(V_{2,SB_2}, V_{3,SB_2}) = 0.5$ | $C(V_{2,SB_3}, V_{3,SB_3}) = -0.5$ | -1/6 |
| $f_2, f_4$ | $C(V_{2,SB_1}, V_{4,SB_1}) = -0.5$ | $C(V_{2,SB_2}, V_{4,SB_2}) = -0.5$ | $C(V_{2,SB_3}, V_{4,SB_3}) = -0.5$ | -1/2 |
| $f_2, f_5$ | $C(V_{2,SB_1}, V_{5,SB_1}) = -0.5$ | $C(V_{2,SB_2}, V_{5,SB_2}) = -0.5$ | $C(V_{2,SB_3}, V_{5,SB_3}) = 0.5$ | -1/6 |
| $f_3, f_4$ | $C(V_{3,SB_1}, V_{4,SB_1}) = 1.0$ | $C(V_{3,SB_2}, V_{4,SB_2}) = 0.5$ | $C(V_{3,SB_3}, V_{4,SB_3}) = -0.5$ | 1/3 |
| $f_3, f_5$ | $C(V_{3,SB_1}, V_{5,SB_1}) = -0.5$ | $C(V_{3,SB_2}, V_{5,SB_2}) = -1.0$ | $C(V_{3,SB_3} V_{5,SB_3}) = -1.0$ | -5/6 |
| $f_4, f_5$ | $C(V_{4,SB_{,1}}, V_{5,SB_1}) = -0.5$ | $C(V_{4,SB_2}, V_{5,SB_2}) = -0.5$ | $C(V_{4,SB_3}, V_{5,SB_3}) = 0.5$ | -1/6 |

In Step S208, the computing module 123 may determine whether the feature $f_A$ and the feature $f_B$ are the selected feature pair according to the threshold value m2 and the relation index $RI(f_A, f_B)$.

In an embodiment, the threshold value m2 may be associated with a relation index ranking of a feature pair in $C_2^K$ feature pairs. For example, the threshold value m2 may indicate that feature pairs with what high relation indexes in the $C_2^K$ feature pairs are used as selected feature pairs. Taking Table 7 as an example, the computing module 123 may select the feature pair $(f_1, f_2)$ with the highest relation index from 10 feature pairs according to the threshold value m2 as the selected feature pair. In other words, the computing module 123 may select the feature pair $(f_1, f_2)$ from the 10 feature pairs in Table 7 as the selected feature pair in response to the relation index $RI(f_1, f_2)$ being greater than the relation indexes $RI(f_1, f_3)$, $RI(f_1, f_4)$, $RI(f_1, f_5)$, $RI(f_2, f_3)$, $RI(f_2, f_3)$, $RI(f_2, f_5)$, $RI(f_3, f_4)$, $RI(f_3, f_5)$, and $RI(f_4,f_5)$.

In an embodiment, the computing module 123 may select the feature pair $(f_A, f_B)$ as the selected feature pair in response to the relation index $RI(f_A, f_B)$ exceeding the threshold value m2. Taking Table 7 as an example, assuming that the threshold value m2 is equal to 1/4, the computing module 123 may select the feature pair $(f_1, f_2)$ as the selected feature pair in response to the relation index $RI(f_1, f_2)$ being greater than 1/4, but the selected feature pair is not limited to one pair.

After executing Step S206 and Step S208 to respectively obtain the selected feature and the selected feature pair, in Step S209, the computing module 123 may obtain a feature corresponding to the selected feature from the selected feature pair as an accompanied feature. For example, after determining that the feature $f_A$ is the selected feature and the feature pair $(f_A, f_B)$ is the selected feature pair, the computing module 123 may select the feature $f_B$ corresponding to the feature $f_A$ from the feature pair $(f_A, f_B)$ as the accompanied feature of the feature $f_A$.

In an embodiment, the accompanied feature may be selected by a professional from the K features according to experience as the accompanied feature.

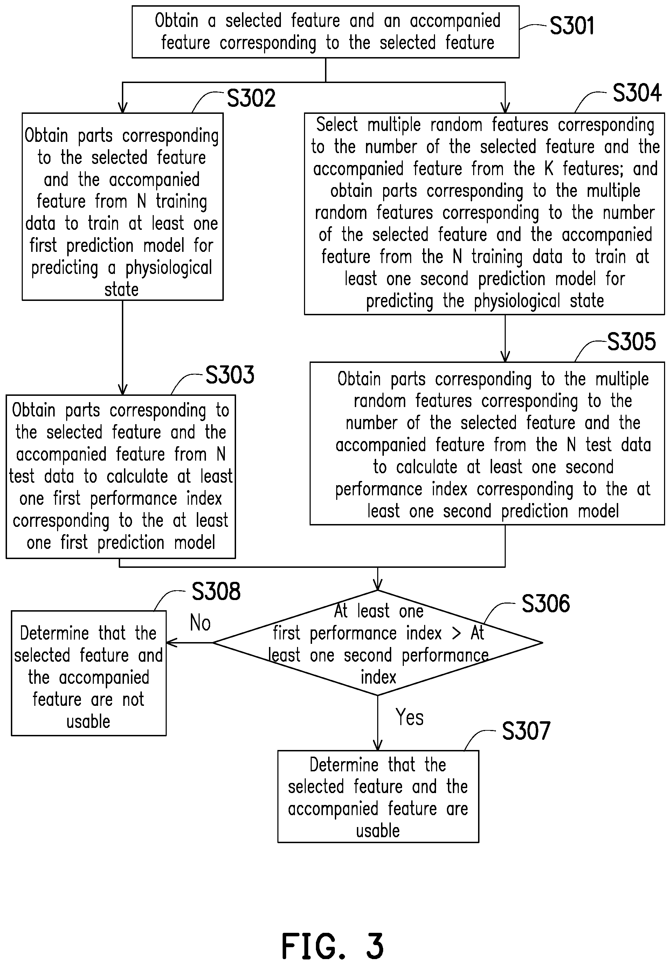

In Step S210, the output module 124 may output the selected feature and the accompanied feature through the transceiver 130. In an embodiment, the computing module 123 may determine whether the selected feature and the accompanied feature are usable. If the selected feature and the accompanied feature are usable, the output module 124 may output the selected feature and the accompanied feature. If the selected feature and the accompanied feature are not usable, the output module 124 may not output the selected feature and the accompanied feature. FIG. 3 is a flowchart of a method for determining whether a selected feature and an accompanied feature are usable according to an embodiment of the disclosure.

In Step S301, the computing module 123 obtains a selected feature and an accompanied feature corresponding to the selected feature.

In Step S302, the training module 122 may obtain parts corresponding to the selected feature and the accompanied feature from N training data to train at least one first prediction model for predicting a physiological state. The at least one first prediction model may correspond to an RF algorithm, a logistic regression, or a SVM, but the disclosure is not limited thereto.

In Step S303, the training module 122 may obtain parts corresponding to the selected feature and the accompanied feature from N test data to calculate at least one first performance index corresponding to the at least one first prediction model. The at least one first performance index may correspond to parameters such as accuracy (ACC), precision, recall rate, false positive (FP), or F1 score in a confusion matrix.

In Step S304, the training module 122 may select two random features from the K features. Any one of the two random features is different from any one of the selected feature and the accompanied feature. Then, the training module 122 may obtain parts corresponding to the two random features from the N training data to train at least one second prediction model for predicting the physiological state. The at least one second prediction model may correspond to the RF algorithm, the logistic regression, or the SVM, but the disclosure is not limited thereto. In an embodiment, the training module 122 may select multiple random features corresponding to the number of selected features and accompanied features from the K features, so as to train the at least one second prediction model. For example, if the total number of selected features and accompanied features obtained by the computing module 123 in Step S301 is 4, the training module 122 may select 4 random features from the K features to train the at least one second prediction model.

In Step S305, the training module 122 may obtain parts corresponding to the two random features (or multiple random features corresponding to the number of selected features and accompanied features) from the N test data to calculate at least one second performance index corresponding to the at least one second prediction model. The at least one second performance index may correspond to the parameters such as ACC, precision, recall, FP, or F1 score in the confusion matrix.

In Step S306, the computing module 123 may determine whether the at least one first performance index is greater than the at least one second performance index. If the at least one first performance index is greater than the at least one second performance index, Step S307 is proceeded. If the at least one first performance index is less than or equal to the at least one second performance index, Step S308 is proceeded.

In Step S307, the computing module 123 may determine that the selected feature and the accompanied feature are usable. In Step S308, the computing module 123 may determine that the selected feature and the accompanied feature are not usable.

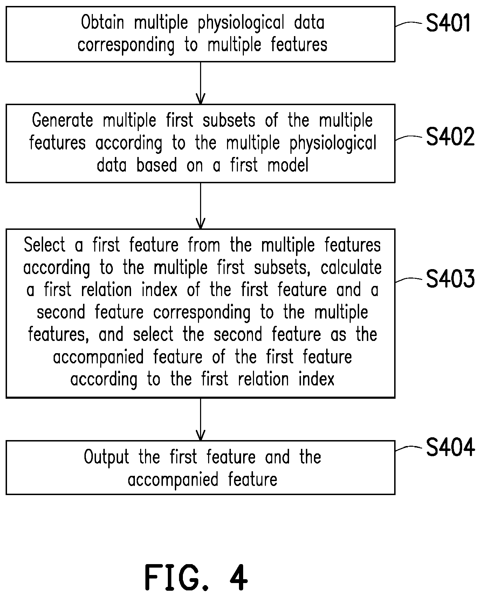

FIG. 4 is a flowchart of a method for screening features for predicting a physiological state according to another embodiment of the disclosure. The method may be implemented by the electronic device 100 shown in FIG. 1. In Step S401, multiple physiological data corresponding to multiple features are obtained. In Step S402, multiple first subsets of the multiple features are generated according to the multiple physiological data based on a first model. The multiple first subsets respectively correspond to the multiple physiological data. In Step S403, a first feature is selected from the multiple features according to the multiple first subsets, a first relation index of the first feature and a second feature corresponding to the multiple features is calculated, and the second feature is selected as the accompanied feature of the first feature according to the first relation index. In Step S404, the first feature and the accompanied feature are output.

In summary, the disclosure may use different types of models to select the feature that significantly affects the prediction result of the physiological state from multiple features, and may select the accompanied feature corresponding to the feature according to the relation index between the feature and other features. The accompanied features selected according to the method may also significantly affect the prediction result of the physiological state. After obtaining at least one feature and at least one corresponding accompanied feature, the disclosure may train the prediction model according to the at least one feature and the at least one accompanied feature, and calculate the performance index of the prediction model. If the performance index shows that the at least one feature and the at least one accompanied feature may significantly affect the prediction result of the physiological state by the prediction model, the disclosure may output the at least one feature and the at least one accompanied feature as reference for the user.

[Description of the Reference Signs]

100: electronic device
110: processor
120: storage medium
121: data collection module
122: training module
123: computing module
124: output module
130: transceiver
S201, S202, S203, S204, S205, S206, S207, S208, S209, S210, S301, S302, S303, S304, S305, S306, S307, S308, S401, S402, S403, S404: Step

**Claims**

1.   An electronic device (100) for screening features for predicting a physiological state, comprising:

a transceiver (130);

a storage medium (120), storing a plurality of modules; and

a processor (110), coupled to the storage medium (120) and the transceiver (130), and accessing and executing the plurality of modules, wherein the plurality of modules comprise:

a data collection module (121), obtaining a plurality of physiological data corresponding to a plurality of features through the transceiver;

a training module (122), generating a plurality of first subsets of the plurality of features according to the plurality of physiological data based on a first model, wherein the plurality of first subsets respectively correspond to the plurality of physiological data;

a computing module (123), selecting a first feature from the plurality of features according to the plurality of first subsets, calculating a first relation index of the first feature and a second feature corresponding to the plurality of features, and selecting the second feature as an accompanied feature of the first feature according to the first relation index; and

an output module (124), outputting the first feature and the accompanied feature through the transceiver (130).

2. The electronic device (100) according to claim 1, wherein

the training module (122) generates a plurality of second subsets of the plurality of features according to the plurality of physiological data based on a second model, wherein the plurality of second subsets respectively correspond to the plurality of physiological data; and

the computing module (123) selects the first feature from the plurality of features according to the plurality of first subsets and the plurality of second subsets.

3. The electronic device (100) according to claim 2, wherein

the computing module (123) calculates a first number of the first feature in the plurality of first subsets, and calculates a second number of the first feature in the plurality of second subsets; and

the computing module (123) selects the first feature from the plurality of features according to the first number and the second number.

4. The electronic device (100) according to claim 3, wherein

the computing module (123) calculates a first score of the first feature according to the first number, a first weight corresponding to the first model, the second number, and a second weight corresponding to the second model; and

the computing module (123) selects the first feature from the plurality of features in response to the first score being greater than a first threshold value.

5. The electronic device (100) according to claim 3, wherein

the computing module (123) calculates a first score of the first feature according to the first number, a first weight corresponding to the first model, the second number, and a second weight corresponding to the second model;

the computing module (123) calculates a third number of a third feature in the plurality of first subsets, and calculates a fourth number of the third feature in the plurality of second subsets;

the computing module (123) calculates a second score of the third feature according to the third number, the first weight, the fourth number, and the second weight; and

the computing module (123) selects the first feature from the first feature and the third feature in response to the first score being greater than the second score.

6. The electronic device (100) according to one of claims 2-5, wherein

the computing module (123) obtains a first number of the first feature in each of the plurality of first subsets to generate a first vector;

the computing module (123) obtains a second number of the second feature in each of the plurality of first subsets to generate a second vector; and

the computing module (123) calculates the first relation index according to the first vector and the second vector.

7. The electronic device (100) according to one of the preceding claims, wherein

the computing module (123) selects the second feature as the accompanied feature of the first feature in response to the first relation index being greater than a second threshold value.

8. The electronic device (100) according to one of the preceding claims, wherein

the computing module (123) calculates a second relation index corresponding to a third feature and a fourth feature in the plurality of features; and

the computing module (123) selects the second feature as the accompanied feature of the first feature in response to the first relation index being greater than the second relation index.

9. The electronic device (100) according to one of the preceding claims, wherein

the training module (122) trains at least one first prediction model of the physiological state according to the plurality of physiological data, the first feature, and the accompanied feature, and calculates at least one first performance index corresponding to the at least one first prediction model;

the training module (122) randomly selects a third feature and a fourth feature from the plurality of features, wherein any one of the third feature and the fourth feature is different from any one of the first feature and the second feature;

the training module (122) trains at least one second prediction model of the physiological state according to the plurality of physiological data, the third feature, and the fourth feature, and calculates at least one second performance index of the at least one second prediction model;

the computing module determines that the first feature and the accompanied feature are usable in response to the at least one first performance index being greater than the at least one second performance index; and

the output module (124) outputs the first feature and the accompanied feature in response to the first feature and the accompanied feature being usable.

10. The electronic device (100) according to one of the preceding claims, wherein the plurality of features respectively correspond to a plurality of types of metabolites of a human body.

11. The electronic device (100) according to claim 9, wherein

the data collection module (121) receives a physiological data set through the transceiver (130), and divides the physiological data set into a plurality of training data and a plurality of test data respectively corresponding to the plurality of physiological data according to a bootstrap;

the training module (122) generates the plurality of first subsets according to the plurality of training data;

the training module (122) generates the at least one first prediction model according to the plurality of training data; and

the training module (122) calculates the at least one first performance index according to the plurality of test data.

12. The electronic device (100) according to one of claims 2-11, wherein the first model or the second model is associated with one of a random forest algorithm, a logistic regression, and a support vector machine.

13. The electronic device (100) according to claim 12, wherein the first model generates the plurality of first subsets based on one of a stepwise selection and a feature importance.

14. A method for screening features for predicting a physiological state, comprising:

obtaining a plurality of physiological data corresponding to a plurality of features;

generating a plurality of first subsets of the plurality of features according to the plurality of physiological data based on a first model, wherein the plurality of first subsets respectively correspond to the plurality of physiological data;

selecting a first feature from the plurality of features according to the plurality of first subsets, calculating a first relation index of the first feature and a second feature corresponding to the plurality of features, and selecting the second feature as an accompanied feature of the first feature according to the first relation index; and

outputting the first feature and the accompanied feature.

**Amended claims in accordance with Rule 137(2) EPC.**

1. An electronic device (100) for screening features for predicting a physiological state, comprising:

a transceiver (130);

a storage medium (120), storing a plurality of modules; and

a processor (110), coupled to the storage medium (120) and the transceiver (130), and accessing and executing the plurality of modules, wherein the plurality of modules comprise:

a data collection module (121), obtaining a plurality of physiological data corresponding to a plurality of features through the transceiver;

a training module (122), generating a plurality of first subsets of the plurality of features according to the plurality of physiological data based on a first model, wherein the plurality of first subsets respectively correspond to the plurality of physiological data;

a computing module (123), selecting a first feature from the plurality of features according to the plurality of first subsets, calculating a first relation index between the first feature and a second feature corresponding to the plurality of features, and selecting the second feature as an accompanied feature of the first feature in response to the first relation index being greater than a threshold value; and

an output module (124), outputting the first feature and the accompanied feature through the transceiver (130), wherein a prediction model for predicting the physiological state is trained according to the first feature and the accompanied feature.

2. The electronic device (100) according to claim 1, wherein

the training module (122) generates a plurality of second subsets of the plurality of features according to the plurality of physiological data based on a second model, wherein the plurality of second subsets respectively correspond to the plurality of physiological data; and

the computing module (123) selects the first feature from the plurality of features according to the plurality of first subsets and the plurality of second subsets.

3. The electronic device (100) according to claim 2, wherein

the computing module (123) calculates a first number of the first feature in the plurality of first subsets, and calculates a second number of the first feature in the plurality of second subsets; and

the computing module (123) selects the first feature from the plurality of features according to the first number and the second number.

4. The electronic device (100) according to claim 3, wherein

the computing module (123) calculates a first score of the first feature according to the first number, a first weight corresponding to the first model, the second number, and a second weight corresponding to the second model; and

the computing module (123) selects the first feature from the plurality of features in response to the first score being greater than a first threshold value.

5. The electronic device (100) according to claim 3, wherein

the computing module (123) calculates a first score of the first feature according to the first number, a first weight corresponding to the first model, the second number, and a second weight corresponding to the second model;

the computing module (123) calculates a third number of a third feature in the plurality of first subsets, and calculates a fourth number of the third feature in the plurality of second subsets;

the computing module (123) calculates a second score of the third feature according to the third number, the first weight, the fourth number, and the second weight; and

the computing module (123) selects the first feature from the first feature and the third feature in response to the first score being greater than the second score.

6. The electronic device (100) according to one of claims 2-5, wherein

the computing module (123) obtains a first number of the first feature in each of the plurality of first subsets to generate a first vector;

the computing module (123) obtains a second number of the second feature in each of the plurality of first subsets to generate a second vector; and

the computing module (123) calculates the first relation index according to the first vector and the second vector.

7. The electronic device (100) according to one of the preceding claims, wherein

the computing module (123) calculates a second relation index corresponding to a third feature and a fourth feature in the plurality of features; and

the computing module (123) selects the second feature as the accompanied feature of the first feature in response to the first relation index being greater than the second relation index.

8. The electronic device (100) according to one of the preceding claims, wherein

the training module (122) trains at least one first prediction model of the physiological state according to the plurality of physiological data, the first feature, and the accompanied feature, and calculates at least one first performance index corresponding to the at least one first prediction model;
the training module (122) randomly selects a third feature and a fourth feature from the plurality of features, wherein any one of the third feature and the fourth feature is different from any one of the first feature and the second feature;
the training module (122) trains at least one second prediction model of the physiological state according to the plurality of physiological data, the third feature, and the fourth feature, and calculates at least one second performance index of the at least one second prediction model;
the computing module determines that the first feature and the accompanied feature are usable in response to the at least one first performance index being greater than the at least one second performance index; and
the output module (124) outputs the first feature and the accompanied feature in response to the first feature and the accompanied feature being usable.

9. The electronic device (100) according to one of the preceding claims, wherein the plurality of features respectively correspond to a plurality of types of metabolites of a human body.

10. The electronic device (100) according to claim 8, wherein

the data collection module (121) receives a physiological data set through the transceiver (130), and divides the physiological data set into a plurality of training data and a plurality of test data respectively corresponding to the plurality of physiological data according to a bootstrap;
the training module (122) generates the plurality of first subsets according to the plurality of training data;
the training module (122) generates the at least one first prediction model according to the plurality of training data; and
the training module (122) calculates the at least one first performance index according to the plurality of test data.

11. The electronic device (100) according to one of claims 2-10, wherein the first model or the second model is associated with one of a random forest algorithm, a logistic regression, and a support vector machine.

12. The electronic device (100) according to claim 11, wherein the first model generates the plurality of first subsets based on one of a stepwise selection and a feature importance.

13. A method for screening features for predicting a physiological state, performed by an electronic device, the method comprising:

obtaining a plurality of physiological data corresponding to a plurality of features;
generating a plurality of first subsets of the plurality of features according to the plurality of physiological data based on a first model, wherein the plurality of first subsets respectively correspond to the plurality of physiological data;
selecting a first feature from the plurality of features according to the plurality of first subsets, calculating a first relation index between the first feature and a second feature corresponding to the plurality of features, and selecting the second feature as an accompanied feature of the first feature in response to the first relation index; and
outputting the first feature and the accompanied feature, wherein a prediction model for predicting the physiological state is trained according to the first feature and the accompanied feature.

FIG. 1

Receive a physiological data set of a test subject —S201

Divide the physiological data set into N physiological data —S202

Generate N subsets SB₁ of the K features according to the N training data based on a first model

Generate N subsets SB₂ of the K features according to the N training data based on a second model

Generate N subsets SB₃ of the K features according to the N training data based on a third model

}S203

Obtain the N subsets SB₁, the N subsets SB₂, and the N subsets SB₃ —S204

Calculate a relation index between each of the K features and other features —S207

Calculate a score of each of the K features according to N subsets SBₘ —S205

Determine whether the feature $f_A$ and the feature $f_B$ are the selected feature pair according to the threshold value m2 and the relation index $RI(f_A, f_B)$ —S208

Determine whether the feature $f_j$ is a selected feature according to a threshold value m1 and the score $Z_j$ —S206

Obtain a feature corresponding to the selected feature from the selected feature pair as an accompanied feature —S209

Output the selected feature and the accompanied feature —S210

# FIG. 2

Obtain a selected feature and an accompanied feature corresponding to the selected feature —S301

Obtain parts corresponding to the selected feature and the accompanied feature from N training data to train at least one first prediction model for predicting a physiological state —S302

Select multiple random features corresponding to the number of the selected feature and the accompanied feature from the K features; and obtain parts corresponding to the multiple random features corresponding to the number of the selected feature and the accompanied feature from the N training data to train at least one second prediction model for predicting the physiological state —S304

Obtain parts corresponding to the selected feature and the accompanied feature from N test data to calculate at least one first performance index corresponding to the at least one first prediction model —S303

Obtain parts corresponding to the multiple random features corresponding to the number of the selected feature and the accompanied feature from the N test data to calculate at least one second performance index corresponding to the at least one second prediction model —S305

At least one first performance index > At least one second performance index —S306

No —S308
Determine that the selected feature and the accompanied feature are not usable

Yes —S307
Determine that the selected feature and the accompanied feature are usable

FIG. 3

```
┌─────────────────────────────────────┐
│  Obtain multiple physiological data  │ ─── S401
│   corresponding to multiple features │
└─────────────────────────────────────┘
                 │
                 ▼
┌─────────────────────────────────────┐
│  Generate multiple first subsets of  │
│  the multiple features according to  │ ─── S402
│   the multiple physiological data    │
│         based on a first model       │
└─────────────────────────────────────┘
                 │
                 ▼
┌─────────────────────────────────────┐
│  Select a first feature from the     │
│  multiple features according to the  │
│  multiple first subsets, calculate   │
│  a first relation index of the first │ ─── S403
│  feature and a second feature        │
│  corresponding to the multiple       │
│  features, and select the second     │
│  feature as the accompanied feature  │
│  of the first feature according to   │
│  the first relation index            │
└─────────────────────────────────────┘
                 │
                 ▼
┌─────────────────────────────────────┐
│   Output the first feature and the   │ ─── S404
│        accompanied feature           │
└─────────────────────────────────────┘
```

# FIG. 4

Europäisches
Patentamt

European
Patent Office

Office européen
des brevets

**EUROPEAN SEARCH REPORT**

Application Number

EP 21 17 5249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HSU YU-HAN H ET AL: "Integrating untargeted metabolomics, genetically informed causal inference, and pathway enrichment to define the obesity metabolome", INTERNATIONAL JOURNAL OF OBESITY, NATURE PUBLISHING GROUP UK, LONDON, vol. 44, no. 7, 28 May 2020 (2020-05-28), pages 1596-1606, XP037166549, ISSN: 0307-0565, DOI: 10.1038/S41366-020-0603-X [retrieved on 2020-05-28] * the whole document, in particular the abstract; section "Materials and methods" on page 1597-1600; section "Results" on pages 1600-1602; first two paragraphs in section "Discussion" on page 1604. * ----- | 1-14 | INV. G16H50/20 G16H50/70 |
| X | US 2020/194126 A1 (LIM RYAN [US] ET AL) 18 June 2020 (2020-06-18) * page 1, paragraph 0002 - page 5, paragraph 0052 * * page 8, paragraph 0098 - page 10, paragraph 0112 * * page 11, paragraph 0118 - page 13, paragraph 0135 * * figures 1-13 * ----- | 1-14 | |
| | | | TECHNICAL FIELDS SEARCHED (IPC) |
| | | | G16H |
| X | US 2019/310269 A1 (CIRULLI ELIZABETH [US] ET AL) 10 October 2019 (2019-10-10) * page 1, paragraph 0002 - page 14, paragraph 0108 * * figures 1-20 * ----- -/-- | 1-14 | |

The present search report has been drawn up for all claims

| Place of search | Date of completion of the search | Examiner |
|---|---|---|
| Munich | 5 November 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 17 5249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Ebbels Timothy M.D.: "Chapter 7 Non-linear Methods for the Analysis of Metabolic Profiles" In: "The Handbook of Metabonomics and Metabolomics", 11 December 2006 (2006-12-11), Elsevier B.V., XP055857619, pages 201-226, * the whole document * | 1-14 | |
| A | Anonymous: "Machine learning - Wikipedia", , 20 December 2020 (2020-12-20), XP055858166, Retrieved from the Internet: URL:https://en.wikipedia.org/w/index.php?title=Machine_learning&oldid=995303079#Model_assessments [retrieved on 2021-11-04] * the whole document * | 1-14 | |
| A | Anonymous: "Metabolomics - Wikipedia", , 22 December 2020 (2020-12-22), XP055857630, Retrieved from the Internet: URL:https://en.wikipedia.org/w/index.php?title=Metabolomics&oldid=995706304 [retrieved on 2021-11-03] * the whole document * | 1-14 | |

TECHNICAL FIELDS
SEARCHED      (IPC)

The present search report has been drawn up for all claims

| Place of search | Date of completion of the search | Examiner |
|---|---|---|
| Munich | 5 November 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 17 5249

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2021

| Patent document cited in search report | | Publication date | Patent family member(s) | | Publication date |
|---|---|---|---|---|---|
| US 2020194126 | A1 | 18-06-2020 | NONE | | |
| US 2019310269 | A1 | 10-10-2019 | US 2019310269 A1 | | 10-10-2019 |
| | | | WO 2019195638 A1 | | 10-10-2019 |

EPO FORM P0459

For more details about this annex : see Official Journal of the European Patent Office, No. 12/82